# FASCICULE DE BREVET EUROPEEN

(11) **EP 3 801 283 B1**
(45) Date de publication et mention de la délivrance du brevet: **13.03.2024**
(21) Numéro de dépôt: 19742435.1
(22) Date de dépôt: 03.06.2019
(51) Int. Cl.: A61B 8/12, A61B 8/08, A61B 8/00

(54) **SONDE ECHOGRAPHIQUE ENDOSCOPIQUE ET GAINE POUR UNE TELLE SONDE**
ENDOSKOPISCHE ULTRASCHALLSONDE UND HÜLLE FÜR EINE SOLCHE SONDE
ENDOSCOPIC ULTRASOUND PROBE AND SHEATH FOR SUCH A PROBE

(30) Priorité: 01.06.2018 FR 1854803
(43) Date de publication de la demande: 14.04.2021
(73) Titulaire: Université Grenoble Alpes, 38400 Saint Martin d'Hères (FR)
(72) Inventeur: CUSTILLON, Guillaume, 38130 Echirolles (FR); DUBOIS, Nicolas, 38330 Montbonnot Saint Martin (FR)
(74) Mandataire: Regimbeau
(86) Numéro de dépôt international: PCT/FR2019/051293
(87) Numéro de publication internationale: WO 2019/229399

(56) Documents cités:
- WO-A1-01/22866
- WO-A1-95/28129
- WO-A1-2009/108863
- US-A- 5 827 313

## Description

### DOMAINE DE L'INVENTION

La présente invention concerne une sonde échographique endoscopique, adaptée pour être utilisée dans le cadre d'une arthroscopie, notamment pour de l'imagerie intra-articulaire per-opératoire minimalement invasive, ainsi qu'une gaine stérile pour une telle sonde.

### ETAT DE LA TECHNIQUE

L'imagerie médicale regroupe les moyens d'acquisition et de restitution d'images à partir de différents phénomènes physiques : résonance magnétique, réflexion d'ondes ultrasonores, radioactivité, absorption des rayons X... Ces technologies permettent de visualiser la physiologie ou le métabolisme du corps humain, d'établir un diagnostic précis et d'orienter les choix thérapeutiques. Cependant, elles représentent une panoplie d'imagerie imparfaite : certaines informations restent inaccessibles ou peu ergonomiques (notamment en n'étant pas en temps réel), et les irradiations sont reconnues comme un problème majeur pour les patients et le personnel soignant.

Seules certaines technologies sont disponibles au bloc opératoire selon le type de chirurgie. L'orthopédie requiert l'identification des structures osseuses et fait donc principalement appel à l'utilisation de rayons X.

L'utilisation de l'imagerie per-opératoire a pour but de donner des informations supplémentaires quant aux tissus lésés, d'augmenter la précision du geste opératoire, de réduire le temps d'intervention dans un contexte de chirurgie mini-invasive qui permet l'augmentation des bénéfices cliniques.

L'arthroscopie est une intervention chirurgicale mini-invasive au cours de laquelle une caméra miniature est introduite dans une articulation par une incision punctiforme et un instrument miniature est introduit par un deuxième orifice punctiforme. Le chirurgien peut donc réaliser un acte diagnostique en appréciant les structures anatomiques de la cavité articulaire et un acte thérapeutique adapté grâce à l'instrument approprié. Cette approche chirurgicale, réalisée en ambulatoire, a révolutionné la pratique chirurgicale en permettant d'opérer sans ouvrir et avec une diminution de la morbidité post-opératoire (diminution de la durée d'hospitalisation et de la période de réadaptation fonctionnelle).

Les articulations du genou et de l'épaule ont rapidement bénéficié de cette innovation chirurgicale. Tout ou partie des structures anatomiques constitutives de ces articulations peuvent être altérées lors d'un traumatisme ou d'une arthrose. Une arthroscopie du genou peut ainsi porter sur les ménisques (par exemple en vue d'une ablation), le cartilage (par exemple en vue d'une régularisation), la synoviale (par exemple pour une excision d'adhérence), la résection de petits fragments cartilagineux ou osseux (corps étrangers), ou certaines chirurgies plus importantes du genou (ligamentoplasties ou autres). De même, et de manière non exhaustive, une indication d'arthroscopie de l'épaule peut être posée pour la prise en charge de calcifications intratendineuses de la coiffe des rotateurs, d'un conflit sous-acromial traité en réalisant une bursectomie et acromioplastie sous arthroscopie, ou encore pour qualifier, en per-opératoire, une rupture de la coiffe des rotateurs.

Lors d'une arthroscopie, l'exploration visuelle de l'intérieur de la cavité articulaire se limite à la surface des structures constitutives de l'articulation, avec, comme conséquences, de potentielles limitations diagnostiques ou thérapeutiques.

Une sonde échographique endoscopique a la capacité d'imager plusieurs structures intra-articulaires (ligaments, cartilage) en profondeur et apporte ainsi des informations supplémentaires par rapport à l'arthroscopie seule, qui permettent au chirurgien d'affiner le diagnostic voire d'adapter le geste opératoire.

Pour pouvoir être utilisée au bloc opératoire, une telle sonde doit être stérile.

Un dispositif médical est dit stérile si la probabilité qu'un micro-organisme viable soit présent est inférieure ou égale à 10⁻⁶ (norme EN 556). La stérilisation est un procédé visant à rendre stérile la charge à stériliser (norme EN 285).

Les méthodes conventionnelles de stérilisation incluent :
- la chaleur humide, dite « à vapeur saturée » en autoclave ;
- les radiations ionisantes (rayons γ, électrons, UV) ;
- la stérilisation plasma utilisant des agents chimiques (par exemple peroxyde d'hydrogène ou acide peracétique).

L'autoclave est la méthode de référence dans le milieu hospitalier ; cependant, elle n'est pas adaptée pour une sonde échographique car celle-ci est sensible à la température.

Ce problème entrave donc l'utilisation d'une telle sonde en routine clinique.

Le document US 5 827 313 A divulgue une gaine pour une sonde échographique endoscopique comprenant une première portion flexible une première portion flexible adaptée pour entourer un cathéter de la sonde échographique, en un matériau adapté pour laisser passer les ultrasons, présentant une extrémité distale fermée.

### EXPOSE DE L'INVENTION

Un but de l'invention est de concevoir une sonde échographique endoscopique qui soit stérile, utilisable dans tout bloc de chirurgie, en procurant des images de qualité suffisante pour permettre de visualiser en profondeur des structures anatomiques.

A cet effet, l'invention propose une gaine pour une sonde échographique endoscopique comprenant :
- une première portion adaptée pour entourer un cathéter de la sonde échographique, en un matériau adapté pour laisser passer les ultrasons, présentant une extrémité distale fermée,
- une seconde portion adaptée pour entourer une poignée et un câble de connexion électrique de la sonde à une station échographique, et
- un raccord agencé entre la première et la deuxième portion, ledit raccord étant adapté pour être fixé de manière amovible à la poignée.

Dans le présent texte, les termes relatifs « proximal » et « distal » s'entendent respectivement d'une partie de la sonde située du côté de l'opérateur qui la manipule, et d'une partie de la sonde du côté du corps du patient dans lequel elle est destinée à être insérée.

De manière particulièrement préférée, la première portion est en un copolymère bloc polyamide-polyether.

De manière avantageuse, la première portion présente une épaisseur constante.

Selon un mode de réalisation, l'épaisseur de la première portion est comprise entre 0,4 et 0,6 mm.

La seconde portion présente avantageusement un diamètre supérieur à celui de la première portion.

Selon un mode de réalisation, le raccord comprend une ouverture centrale pour le passage du cathéter, la première portion étant fixée autour de ladite ouverture centrale du raccord et la seconde portion étant fixée à une portion périphérique du raccord.

Selon une forme d'exécution, le raccord est adapté pour être fixé à la poignée par un mécanisme de type quart de tour.

Selon une autre forme d'exécution, le raccord est adapté pour être fixé à la poignée par encliquetage.

L'invention concerne également une sonde échographique endoscopique, comprenant :
- un capteur échographique,
- un connecteur adapté pour être connecté à une station échographique,
- une poignée de commande,
- un câble de connexion électrique s'étendant entre le connecteur et la poignée,
- un cathéter comprenant une portion proximale fixée à la poignée et une portion distale dans laquelle est agencé le capteur échographique,
ladite sonde étant caractérisée en ce qu'elle comprend une gaine stérilisable telle que décrite plus haut, la première portion de la gaine entourant le cathéter, la seconde portion entourant la poignée et le câble, et le raccord étant fixé de manière amovible à la poignée.

Selon un mode de réalisation, le capteur échographique est agencé en regard d'une lentille située sur une paroi du cathéter.

La première portion de la gaine recouvre la portion du cathéter dans laquelle est agencé le capteur échographique.

Selon un mode de réalisation, le cathéter présente une portion distale inclinée et/ou une extrémité distale biseautée. Dans ce cas, la lentille est avantageusement agencée à l'extrémité distale du cathéter.

De manière alternative, la lentille est agencée sur la circonférence du cathéter.

Un autre objet de l'invention est un système d'imagerie échographique comprenant une station échographique et une sonde échographique endoscopique telle que décrite ci-dessus reliée à ladite station par le connecteur, dans lequel la station comprend un processeur configuré pour traiter une image d'une zone d'intérêt intra-articulaire acquise par la sonde et pour déterminer, à partir de ladite image, au moins une des informations suivantes :
- une épaisseur de cartilage ;
- une rupture du ligament croisé antérieur ;
- un emplacement d'un point d'insertion du ligament croisé antérieur.

### DESCRIPTION DES FIGURES

D'autres caractéristiques et avantages de l'invention ressortiront de la description détaillée qui va suivre, en référence aux dessins annexés sur lesquels :
- la figure 1 est une vue en perspective d'une sonde selon l'invention ;
- la figure 2 est une vue en perspective d'une sonde selon un mode de réalisation dans lequel la gaine est fixée par clip sur la poignée ;
- la figure 3 est une vue en perspective de la gaine de la figure 2 avant sa fixation sur la poignée ;
- la figure 4 est une vue en perspective d'une sonde selon un mode de réalisation dans lequel la gaine est fixée par quart de tour sur la poignée ;
- la figure 5 est une vue en perspective de la gaine de la figure 4 avant sa fixation sur la poignée ;
- la figure 6 est une vue schématique d'un premier montage de test qualitatif de la gaine ;
- les figures 7A-7D sont des images échographiques obtenues grâce au montage de la figure 6, respectivement en l'absence de gaine, avec une gaine en PEBD, avec une gaine en PEBAX^{™} 2533 et avec une gaine en PEBAX^{™} 3533;
- la figure 8 est une vue schématique d'un second montage de test qualitatif de la gaine ;
- la figure 9 est un graphique représentant l'atténuation globale (en dB) calculée par intégration des atténuations de la figure 9 sur l'ensemble des fréquences pour les différents matériaux testés ;
- les figures 10A et 10B sont des images échographiques d'un cartilage animal épais obtenues par une sonde respectivement dépourvue de gaine et protégée par une gaine en PEBAX^{™} 2533 ;
- les figures 11A et 11B sont des images échographiques d'un cartilage mince obtenues par une sonde respectivement dépourvue de gaine et protégée par une gaine en PEBAX^{™} 2533.

Les signes de référence identiques d'une figure à l'autre désignent des éléments identiques ou remplissant la même fonction.

### DESCRIPTION DETAILLEE DE MODES DE REALISATION DE L'INVENTION

La sonde échographique endoscopique (également appelée sonde endo-échographique) fait partie d'un système d'imagerie comprenant une station d'échographie à laquelle la sonde est reliée.

La station comprend une électronique de pilotage échographique, un processeur adapté pour traiter les images acquises par la sonde, un écran de visualisation et un clavier permettant d'entrer des données et d'annoter les images.

Pendant une opération sous arthroscopie de l'épaule ou du genou, la sonde endo-échographique est insérée dans l'articulation en utilisant la voie instrumentale. La sonde est toujours utilisée avec l'arthroscope qui utilise l'autre voie. Le chirurgien amène la partie distale de la sonde dans la zone d'intérêt en s'aidant de l'arthroscope et inspecte les structures anatomiques dans cette zone en orientant la sonde vers celles-ci.

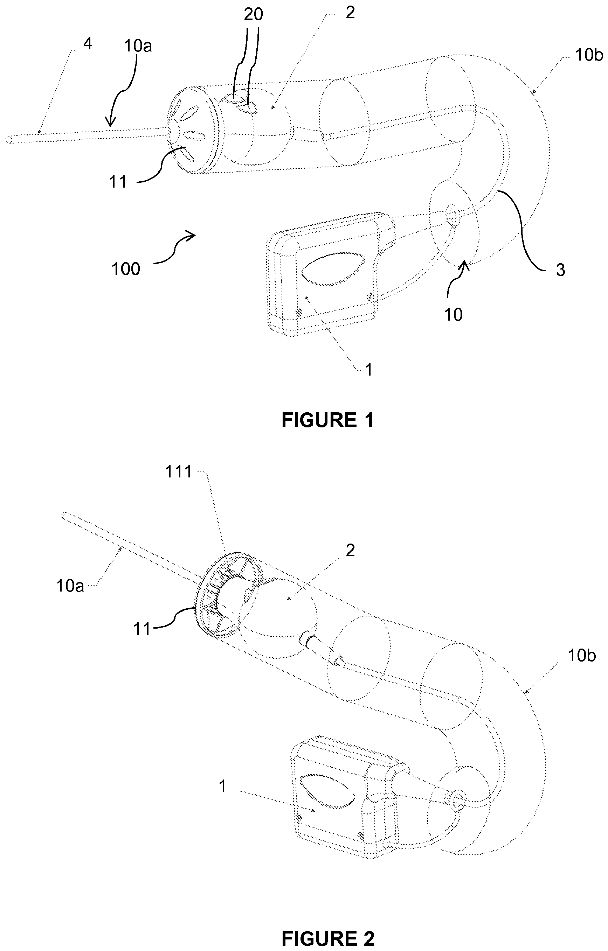

La figure 1 est une vue en perspective d'une sonde selon l'invention.

La sonde 100 comprend un connecteur 1 par lequel elle peut être connectée à la station d'échographie (qui n'est pas représentée).

Le connecteur 1 est relié à une poignée 2 de commande par l'intermédiaire d'un câble 3 de connexion électrique. La poignée de commande est l'organe que l'utilisateur tient dans sa main lors de l'intervention. La poignée comprend un ou plusieurs boutons 20 que l'utilisateur peut presser par exemple pour naviguer ou pour valider l'acquisition d'une image ou d'une vidéo.

A partir de la poignée 2 s'étend un cathéter 4 dans lequel est agencé un capteur échographique (non représenté). Le cathéter présente une longueur généralement comprise entre 10 et 20 cm et un diamètre de quelques millimètres, par exemple 4 mm. Le cathéter peut être en inox ou en titane.

Bien que le cathéter soit représenté droit sur les figures annexées, il peut également présenter une portion courbée et/ou une extrémité distale biseautée. Par « portion courbée » on entend que la portion distale du cathéter est inclinée par rapport à la portion proximale du cathéter, lesdites portions étant reliées par une portion courbée. Par « extrémité distale biseautée » on entend que le plan de l'extrémité distale est incliné par rapport à l'axe de révolution du cathéter. Ces inclinaisons sont typiquement de l'ordre d'une dizaine à une trentaine de degrés. Une telle configuration du cathéter est particulièrement avantageuse en arthroscopie, car elle permet de mieux s'adapter à la géométrie de l'articulation à inspecter. Ainsi, par exemple, le cathéter peut présenter une portion distale inclinée de 10° par rapport à la portion proximale du cathéter, et une extrémité biseautée d'un angle de 20° par rapport à l'axe de révolution du cathéter.

De manière avantageuse, le capteur échographique est situé en regard d'une lentille (non illustrée) agencée dans la surface circonférentielle du cathéter, dans la portion distale du cathéter. De manière alternative, la lentille peut être agencée à l'extrémité distale du cathéter, notamment lorsque le cathéter est courbé ou biseauté comme mentionné ci-dessus.

Le capteur comprend avantageusement un transducteur multi-éléments agencés sous la forme d'un réseau linéaire, par exemple au nombre de 64 éléments. Le capteur présente par exemple une longueur de 13 mm et la lentille une longueur de 16,5 mm, le capteur étant centré par rapport à la lentille.

La sonde et le fonctionnement de la station d'échographie en tant que tels sont connus et ne seront donc pas décrit en détail dans le présent texte.

La sonde est protégée par une gaine stérile 10 qui la recouvre entièrement, de l'extrémité distale du cathéter jusqu'au connecteur. La gaine est d'un seul tenant et ne présente une ouverture qu'au niveau du connecteur 1.

La gaine présente une première partie 10a adaptée pour entourer le cathéter, et une seconde partie 10b, plus large, entourant la poignée et le câble de connexion. Les dimensions de la gaine sont choisies pour s'adapter à la forme et aux dimensions de la sonde. D'une manière générale, la première et la seconde partie présentent chacune une forme cylindrique de section circulaire.

La première partie 10a épouse la forme du cathéter 4 au plus près afin de ne pas augmenter excessivement l'encombrement du cathéter, un léger jeu étant ménagé pour faciliter l'enfilage de la gaine sur le cathéter. Par exemple, pour une gaine de 0,5 mm d'épaisseur et un cathéter de 4 mm de diamètre, le diamètre extérieur de la première portion est de l'ordre de 5,4 mm. La première portion 10a de la gaine est fermée à son extrémité distale, y compris en regard de la lentille. La première portion 10a est suffisamment flexible compte tenu de son matériau et de son épaisseur pour s'adapter à la forme du cathéter, que celui soit droit ou courbé.

La première et la seconde partie de la gaine sont reliées par un raccord 11 grâce auquel la gaine peut être fixée de manière amovible à la poignée de la sonde.

Le raccord est typiquement en un matériau thermoplastique.

La première portion 10a de la gaine est en un matériau adapté pour laisser passer le signal échographique avec une atténuation suffisamment faible pour que l'imagerie soit réalisable avec une résolution suffisante. Le choix d'un matériau adapté pour cette fonction sera décrit en détail plus bas. De préférence, la première portion de la gaine est en un copolymère bloc polyamide-polyether commercialisé notamment sous la dénomination PEBAX^{™}, ou en PEBD. De préférence, le PEBAX^{™} 2533 est préféré, le PEBAX^{™} 3533 étant satisfaisant mais moins préféré. L'épaisseur de la première portion de la gaine est choisie suffisamment fine pour ne pas engendrer une atténuation trop importante des ultrasons. Par exemple, l'épaisseur de cette première portion est comprise entre 0,4 et 0,6 mm, de manière préférée 0,5 mm. Cette épaisseur est avantageusement constante sur toute la longueur de la première portion.

La seconde portion 10b de la gaine peut être réalisée dans le même matériau que la première portion ou dans un autre matériau, cette seconde portion n'étant pas destinée à laisser passer les ultrasons. La seconde portion ne comporte pas nécessairement de moyen de fixation au connecteur. Elle est en effet suffisamment longue pour recouvrir la sonde jusqu'à la zone non stérile du site opératoire, même si elle est simplement enfilée autour du câble de connexion.

Le raccord 11 présente une ouverture centrale 110 pour le passage du cathéter 4, autour de laquelle il s'étend radialement, la première portion 10a de la gaine étant fixée à une portion centrale du raccord, autour de ladite ouverture 110, et s'étend du côté distal du raccord ; la seconde portion 10b de la gaine est fixée à une portion périphérique du raccord et s'étend du côté proximal du raccord. La première et la deuxième portion 10a, 10b sont fixées de manière étanche au raccord 11 par tout moyen approprié, par exemple par collage sur le raccord ou par surmoulage du raccord.

Le raccord 11 et la poignée 2 présentent avantageusement des moyens mutuels de solidarisation réversible. Selon un mode de réalisation, la solidarisation est effectuée par un mécanisme quart de tour. De manière alternative, la solidarisation peut être effectuée par encliquetage.

Le(s) matériau(x) formant les deux portions de la gaine et le raccord sont stérilisables par des radiations ionisantes sans se dégrader. De préférence, la stérilisation est effectuée par une exposition à de l'oxyde d'éthylène suivie par une exposition à des rayons γ.

La gaine 10 étant amovible vis-à-vis du reste de la sonde et recouvrant entièrement la partie de la sonde située dans le champ opératoire, elle peut être stérilisée isolément et permet de procurer un dispositif échographique endoscopique stérile sans risquer d'endommager les composants fonctionnels de la sonde. Typiquement, la gaine est stérilisée avant d'être emballée dans un emballage adapté pour préserver la stérilité et n'est extraite de cet emballage que pour être mise en place sur la sonde.

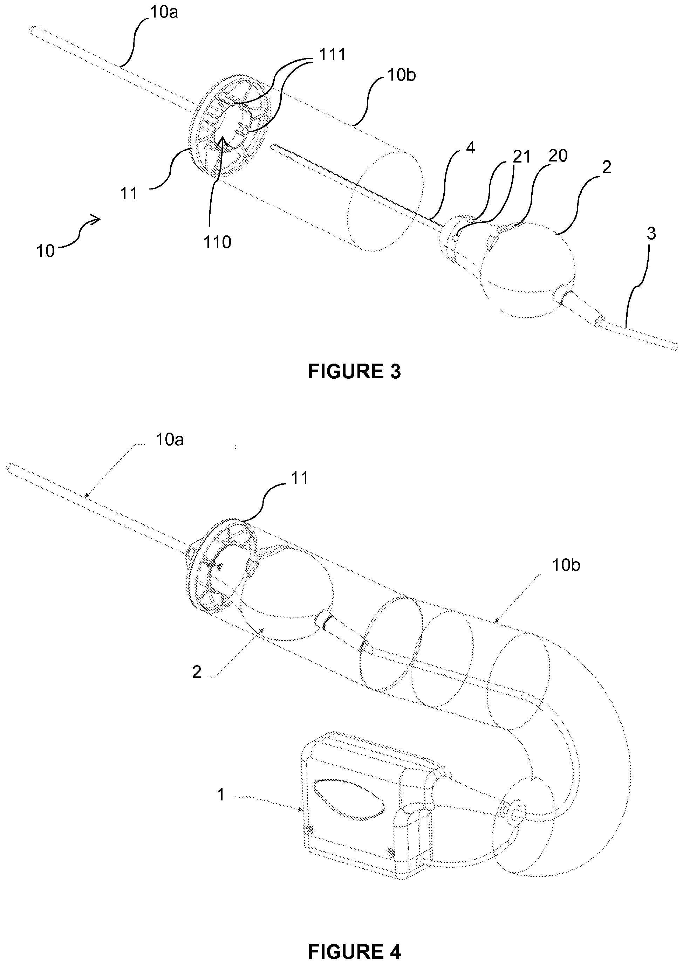

Les figures 2 et 3 illustrent un premier mode de réalisation de la sonde, respectivement après et avant solidarisation de la gaine sur la poignée.

Dans ce mode de réalisation, la fixation est assurée par encliquetage. A cet effet, le raccord 11 comprend des clips 111 qui s'étendent à partir de sa surface proximale, par exemple répartis à intervalles réguliers sur un cercle concentrique à l'axe du cathéter, et la poignée 2 comprend sur sa surface externe des renfoncements 21 correspondants. Après assemblage de la première portion de la gaine sur le cathéter, les clips 111 s'engagent dans les renfoncements 21 correspondants et maintiennent ainsi le raccord 11 sur la poignée 2.

Naturellement, la forme du raccord, de la poignée et des moyens de fixation respectifs pourront varier sans pour autant sortir du cadre de la présente invention.

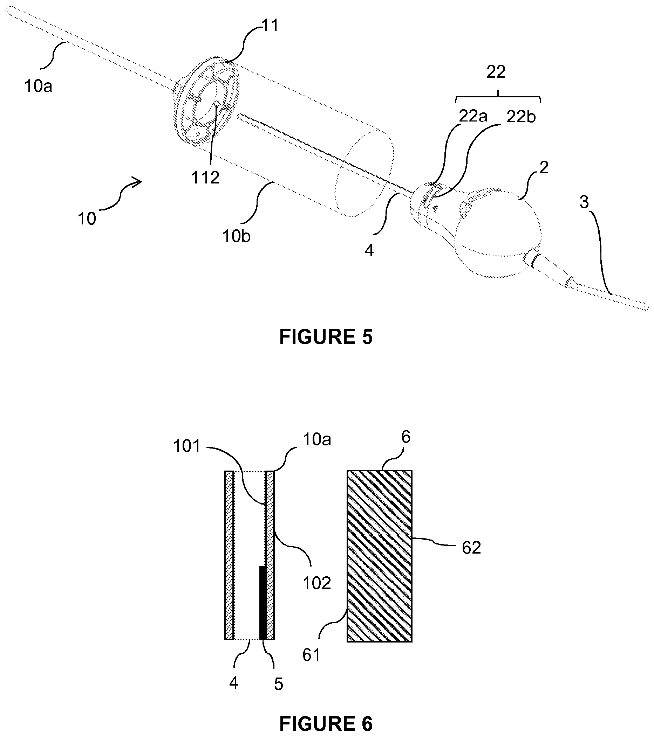

Les figures 4 et 5 illustrent un deuxième mode de réalisation de la sonde, respectivement après et avant solidarisation de la gaine sur la poignée.

Dans ce mode de réalisation, la fixation est assurée par un mécanisme quart de tour. A cet effet, le raccord 11 comprend deux ergots 112 en saillie radiale vers l'intérieur, de préférence radialement opposés, et la poignée 2 comprend deux rainures correspondantes agencées dans sa surface externe. Chaque rainure 22 comprend une portion d'entrée 22a axiale et une portion 22b de maintien s'étendant circonférentiellement à partir de l'extrémité proximale de la portion d'entrée 22a. Après assemblage de la première portion 10a de la gaine sur le cathéter 4, les ergots 112 s'engagent dans les portions d'entrée axiales 22a des rainures jusqu'à arriver en butée à leur extrémité proximale, puis dans les portions de maintien 22b sous l'effet d'un pivotement relatif de la poignée et du raccord.

Ces deux modes de réalisation ont été présentés car ils constituent un moyen particulièrement simple et sûr pour fixer de manière amovible le raccord sur la poignée. Cependant, l'homme du métier pourra choisir tout autre moyen de solidarisation réversible adapté (par exemple vissage, friction, etc.) sans pour autant sortir du cadre de la présente invention.

On va maintenant décrire les modalités de choix du matériau de la première portion de la gaine.

Les inventeurs ont testé différents matériaux transparents aux ondes ultrasonores et les ont évalués qualitativement et quantitativement pour vérifier leur capacité à laisser passer le signal ultrasonore avec une atténuation suffisamment faible.

La figure 6 est un schéma de principe d'un premier montage pour un test qualitatif.

Le capteur échographique 5 de la sonde est agencé en regard d'une paroi 6 en PVC, l'ensemble étant immergé dans de l'eau.

Une première image échographique est acquise sans la gaine, puis une gaine 10a constituée de chacun des matériaux à tester est enfilée sur le cathéter 4, et une image échographique respective est acquise.

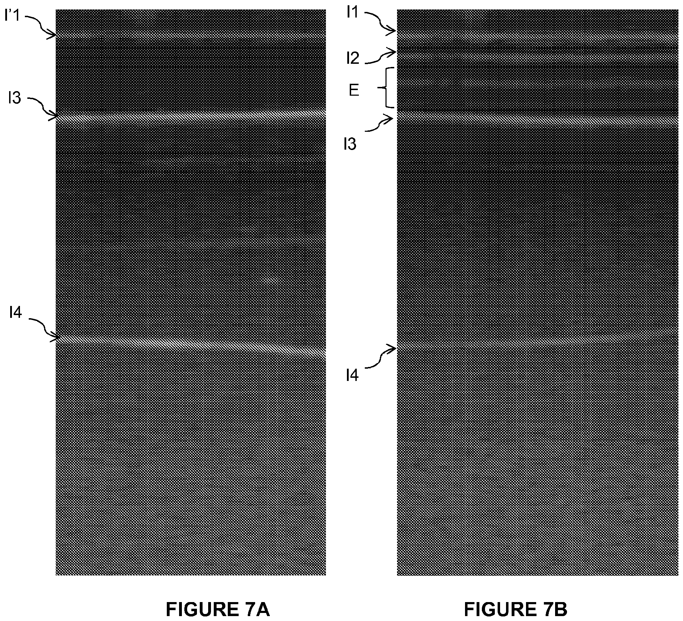

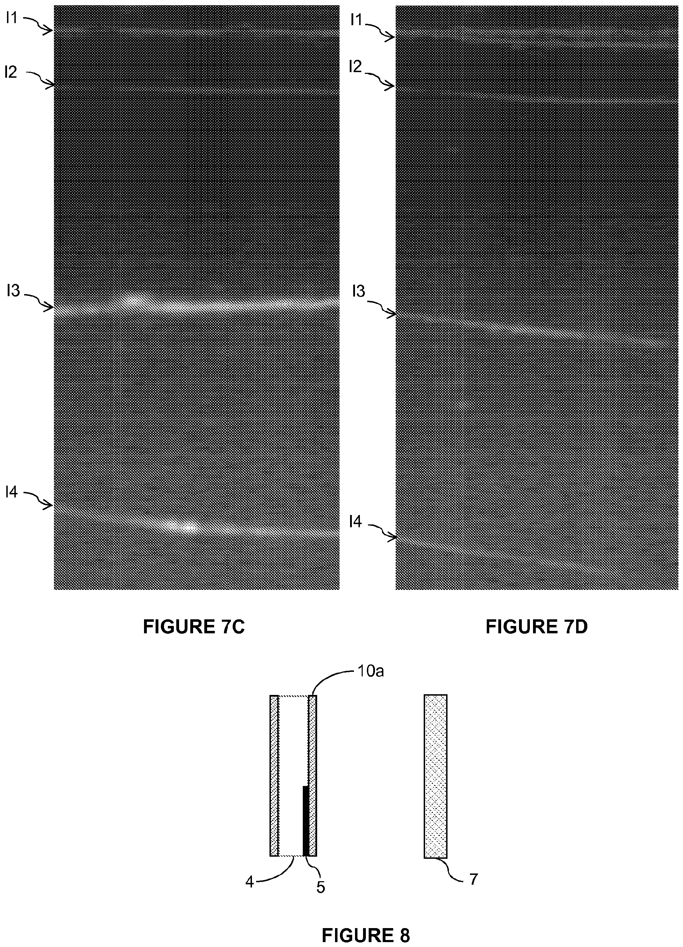

Les résultats présentés sur les figures 7A-7D sont respectivement obtenus sans gaine, avec une gaine en PEBD, avec une gaine en PEBAX^{™} 2533 (matériau préféré) et en PEBAX^{™} 3533. Les lignes blanches désignées par 11, I2, I3 et I4 correspondent respectivement à l'interface entre la lentille et la gaine, l'interface entre la gaine et l'eau, l'interface entre l'eau et le PVC (surface de la paroi en regard de la sonde) et l'interface entre le PVC (surface de la paroi opposée à la sonde) et l'eau. Dans le cas de la figure 7A, en l'absence de gaine, l'interface entre la lentille et l'eau est désignée par l'1.

Un premier critère de qualification d'un matériau envisagé pour la gaine est la visibilité de ces interfaces. On cherche à obtenir une visibilité aussi proche que possible que la visibilité obtenue sans gaine. La perte de visibilité des interfaces 61, 62 de la paroi PVC est due à l'absorption des ultrasons dans la gaine et à l'énergie réfléchie par les surfaces intérieure 101 et extérieure 102 de la gaine.

Par ailleurs, les éventuelles lignes désignées par E correspondent à des interfaces parasites dues à un phénomène de cavité dans l'épaisseur de la gaine. Ce phénomène est causé par une différence d'impédance acoustique entre la gaine et l'eau. De ce fait, les ultrasons font des allers-retours multiples entre la surface externe de la gaine et la sonde, chaque aller-retour produisant un écho qui se traduit par une ligne horizontale sur l'image. De telles interfaces parasites nuisent à la qualité de l'image. Ces échos parasites sont d'autant plus problématiques que dans l'application intra-articulaire visée, la sonde doit être très proche des tissus à échographier, voire au contact de ceux-ci, ce qui implique que les éventuels échos parasites se superposeront aux structures anatomiques à observer.

La présence et le nombre d'échos parasites constitue donc un deuxième critère de qualification du matériau. De tels échos doivent être évités ou tout au moins minimisés.

On observe sur ces images que la gaine en PEBD produit des échos parasites, bien que les interfaces attendues présentent une bonne visibilité. La gaine en PEBAX^{™} 2533, qui est le matériau le plus performant, ne génère pas d'échos parasites tout en procurant des interfaces bien visibles. Le PEBAX^{™} 3533 apparaît également comme un matériau relativement satisfaisant.

Le tableau ci-dessous récapitule les matériaux testés et leur classification en termes de nombre d'échos parasites et de visibilité des interfaces.

| Matériau de la gaine | Nombre d'échos parasites | Visibilité des interfaces |
|---|---|---|
| Sans gaine | 0 | +++ |
| PA12 | 4 | + |
| PEBD | 2 | ++ |
| PVC | 2 | + |
| Silicone | 0 | - |
| TPU95 | 2 | - |
| TPU98 | 3 | - |
| PEBAX^{™} 2533 | 0 | ++ |
| PEBAX^{™} 3533 | 0 | + |
| PEBAX^{™} 4533 | 0 | - |

Une autre étude a été menée pour évaluer quantitativement l'atténuation causée par les différents matériaux envisagés pour la gaine.

La figure 8 est un schéma de principe d'un second montage pour un test qualitatif.

Le capteur échographique 5 de la sonde est agencé en regard d'une paroi 7 en métal, qui est un bon réflecteur des ultrasons, l'ensemble étant fixé par un banc pour maintenir une distance constante entre la sonde et la paroi métallique.

Une première image échographique est acquise sans la gaine, puis une gaine 10a constituée de chacun des matériaux à tester est enfilée sur le cathéter 4, et une image échographique respective est acquise. Un calcul d'atténuation est alors réalisé.

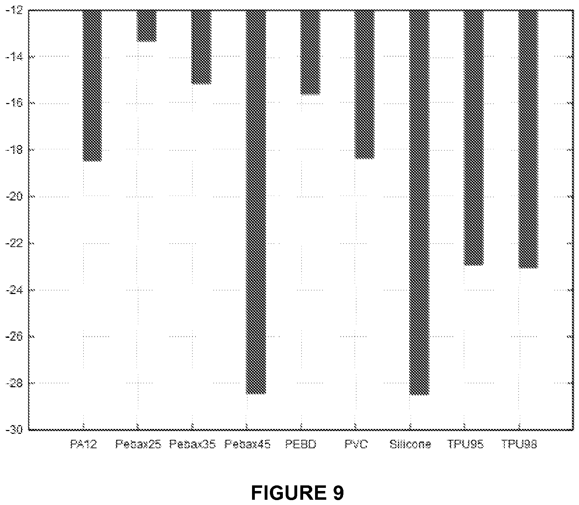

La figure 9 est un graphique représentant l'atténuation globale (c'est-à-dire intégrée sur l'ensemble de la plage de fréquence d'intérêt, qui est comprise entre 10 et 15 MHz) (en dB) pour les différents matériaux testés.

On constate que le silicone et le PEBAX^{™} 4533 présentent une forte absorption, et ne sont donc pas préférés, bien qu'ils ne génèrent pas d'échos parasites. Le PEBAX^{™} 2533 est le meilleur candidat, avec une absorption d'environ 13 dB, suivi du PEBAX^{™} 3533 (15 dB) et du PEBD (15,5 dB). Cependant, le PEBD ne sera pas retenu en raison des échos parasites qu'il génère.

En conclusion, le PEBAX^{™} 2533 est le matériau testé qui présente le moins d'atténuation. De plus, il est important de noter que les gaines en PEBAX^{™} testées étaient plus épaisses (environ 2 mm d'épaisseur) que les autres (environ 0,8 mm). Ainsi, dans des conditions d'épaisseur identiques, une gaine en PEBAX^{™} 2533 présenterait encore moins d'atténuation que les autres.

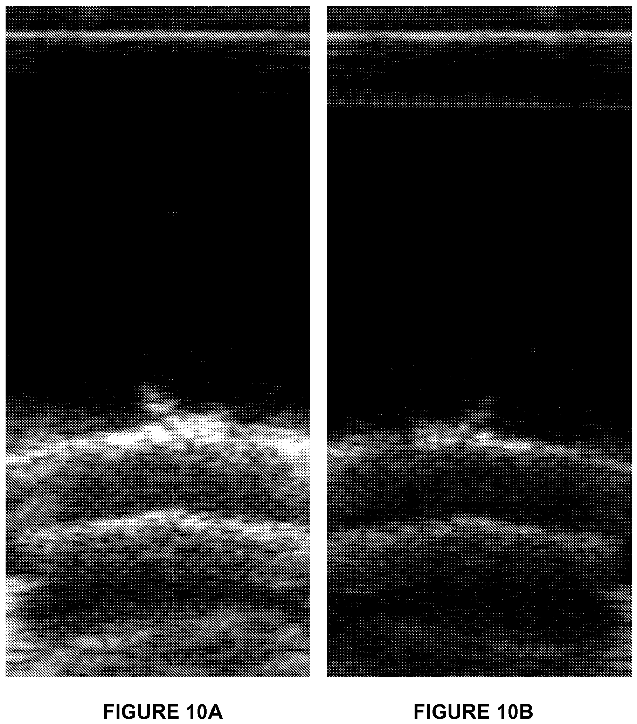

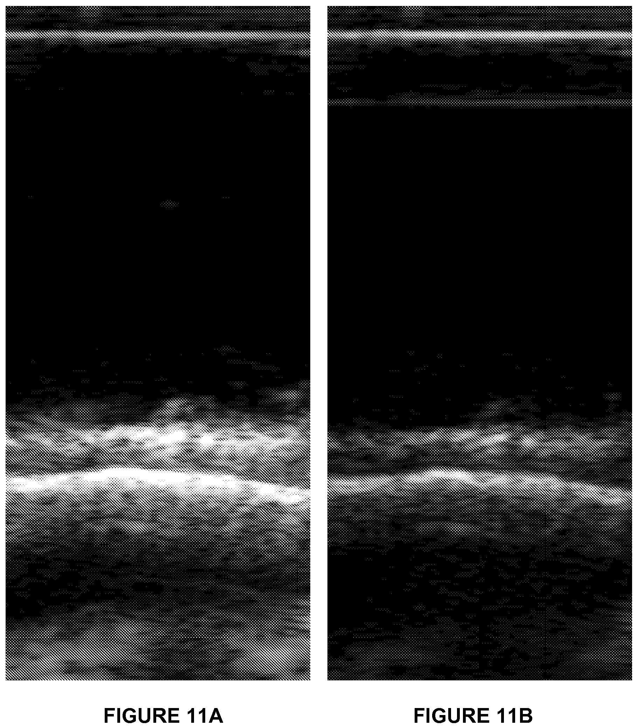

Pour valider que le PEBAX^{™} 2533 correspond bien aux attentes, des images de cartilage ont été réalisées en maintenant fixe la sonde sur des pièces anatomiques animales. Toutes les images ont été enregistrées avec les mêmes réglages de paramètres. Des images de cartilage épais (c'est-à-dire de 2,6 mm d'épaisseur environ) (cf. figures 10A-10B) et mince (c'est-à-dire de 1,6 mm d'épaisseur environ) (cf. figures 11A-11B) ont été enregistrées (visibles dans la partie inférieure des images). Les interfaces délimitant le cartilage sont toujours visibles avec la gaine en PEBAX^{™} 2533.

Lorsque la sonde est à nu dans l'eau, les interfaces sont très brillantes et saturent l'image : il faudrait diminuer le gain pour les observer correctement. Avec la gaine en PEBAX^{™} 2533, la saturation disparaît, comme si le gain avait été diminué à un niveau acceptable.

D'un point de vue acoustique, le PEBAX^{™} 2533 apparaît donc comme le meilleur matériau : son impédance acoustique est proche de celle de l'eau (pas d'échos parasites dus au phénomène de cavité) et il absorbe peu les ultrasons. Par conséquent, les images obtenues au travers de cette gaine présentent une qualité suffisante pour une visualisation en profondeur des structures anatomiques d'intérêt, tout en assurant la stérilité de la sonde.

De plus, le PEBAX^{™} 2533 est un matériau translucide, au travers duquel on peut voir l'intérieur de la sonde, notamment la position du capteur échographique sur les images arthroscopiques, ce qui permet de faciliter l'utilisation de la sonde par un chirurgien.

Grâce à ladite sonde, l'imagerie échographique endoscopique permet en particulier :
- de voir des lésions tissulaires (rupture ligamentaire partielle, calcification intratendineuse, autres) non affleurantes à la surface, par conséquent avec une meilleure localisation;
- d'identifier, à l'aide de nouveaux paramètres, les remaniements/altérations pathologiques tissulaires, comme cela a déjà été démontré, par exemple, dans le cadre de la maladie arthrosique (modifications des propriétés d'échogénicité du cartilage, aux interfaces) ;
- d'estimer l'épaisseur du cartilage à partir de la corrélation entre le temps de vol des ultrasons et l'épaisseur réelle mesurée à l'IRM du cartilage ;
- de faciliter la réalisation de gestes arthroscopiques de résection de calcifications (visibles à l'échographie, mais pas à l'œil), de ténotomie ou de ténodèse suite à une meilleure estimation ultrasonore de l'état inflammatoire du tendon, etc.

L'invention présente un intérêt particulier dans le traitement des lésions du cartilage et du ligament croisé antérieur (ci-après désigné par l'acronyme LCA) dans le genou.

Dans le genou, le cartilage hyalin n'est pas vascularisé et les cellules sont nourries par le liquide synovial qui permet le passage des nutriments à travers le cartilage. Ainsi, la mobilisation de l'articulation est nécessaire pour éviter souffrance et altération du cartilage. De plus, le cartilage ne se restaure pas et sa cicatrisation génère un tissu fibreux irrégulier qui ne procure aucune qualité biomécanique. En cas de lésion, cette cicatrisation entraîne alors de l'arthrose secondaire. L'arthrose primitive est quant à elle due à une usure naturelle du cartilage.

La sonde permet une évaluation per-opératoire des lésions du cartilage du genou à traiter. Elle permet un nettoyage de la lésion afin de déterminer très précisément le dimensionnement en surface et en profondeur, de la surface à traiter et permet ainsi au chirurgien un choix thérapeutique adapté à chacun de ses patients. Par ailleurs, la précision de sa technologie offre la possibilité de différencier le fibrocartilage du cartilage hyalin très recherché pour l'évaluation des produits de thérapies cellulaires.

Pour les pathologies de la gonarthrose, l'évaluation de l'état du cartilage et notamment de l'arthrose précoce associée à une lésion méniscale par exemple peut permettre d'adapter la reprise d'activités physiques en vue de protéger le cartilage.

Le positionnement du tunnel fémoral lors des reconstructions partielles du LCA est crucial : le mauvais positionnement peut mener à l'apparition de symptômes d'instabilité antérieure ou rotatoire ou à une perte de fonction. Des structures anatomiques de référence comme le « resident's ridge » permettent de repérer le placement du tunnel : l'endo-échographie permet de visualiser cette enthèse du LCA.

L'invention présente également un intérêt particulier dans le traitement de la tendinite calcifiante dans l'épaule.

La littérature montre que l'association d'un nettoyage complet des calcifications combiné à une préservation du tendon optimise les suites opératoires des patients.

L'utilisation de la sonde échographique intra-articulaire permet de mieux localiser les calcifications en évitant le geste de trituration. La sonde offre plus de précision dans le geste chirurgical en repérant les calcifications situées à l'intérieur même du tendon : elle repère la calcification et renvoie une image lors de la détection de la lésion calcifiante.

## Revendications

1. Gaine (10) pour une sonde échographique endoscopique, comprenant :
- une première portion (10a) flexible adaptée pour entourer un cathéter (4) de la sonde échographique, en un matériau adapté pour laisser passer les ultrasons, présentant une extrémité distale fermée,
**caractérisée en ce que** ladite gaine (10) comprend en outre
- une seconde portion (10b) adaptée pour entourer une poignée (2) et un câble (3) de connexion électrique de la sonde à une station échographique, et
- un raccord (11) agencé entre la première et la deuxième portion (10a, 10b), ledit raccord (11) étant adapté pour être fixé de manière amovible à la poignée (2).

2. Gaine selon la revendication 1, dans laquelle la première portion (10a) est en un copolymère bloc polyamide-polyether.

3. Gaine selon l'une des revendications 1 à 2, dans laquelle la première portion (10a) présente une épaisseur constante.

4. Gaine selon l'une des revendications 1 à 3, dans laquelle l'épaisseur de la première portion (10a) est comprise entre 0,4 et 0,6 mm.

5. Gaine selon l'une des revendications 1 à 4, dans laquelle la seconde portion (10b) présente un diamètre supérieur à celui de la première portion (10a).

6. Gaine selon l'une des revendications 1 à 5, dans laquelle le raccord comprend une ouverture centrale pour le passage du cathéter, la première portion étant fixée autour de ladite ouverture centrale du raccord et la seconde portion étant fixée à une portion périphérique du raccord.

7. Gaine selon l'une des revendications 1 à 6, dans laquelle le raccord (11) est adapté pour être fixé à la poignée (2) par un mécanisme de type quart de tour.

8. Gaine selon l'une des revendications 1 à 6, dans laquelle le raccord est adapté pour être fixé à la poignée par encliquetage.

9. Sonde échographique endoscopique (100), comprenant :
- un capteur échographique (5),
- un connecteur (1) adapté pour être connecté à une station échographique,
- une poignée (2) de commande,
- un câble (3) de connexion électrique s'étendant entre le connecteur (1) et la poignée (2),
- un cathéter (4) comprenant une portion proximale fixée à la poignée (2) et une portion distale dans laquelle est agencé le capteur échographique,
ladite sonde étant **caractérisée en ce qu'**elle comprend une gaine (10) selon l'une des revendications 1 à 8, la première portion (10a) de la gaine entourant le cathéter (4), la seconde portion (10b) entourant la poignée (2) et le câble (3), et le raccord (11) étant fixé de manière amovible à la poignée (2).

10. Sonde selon la revendication 9, dans laquelle le capteur échographique est agencé en regard d'une lentille située sur une paroi du cathéter.

11. Sonde selon la revendication 10, dans laquelle la première portion (10a) de la gaine recouvre la portion du cathéter dans laquelle est agencé le capteur échographique.

12. Sonde selon l'une des revendications 9 à 11, dans laquelle le cathéter présente une portion distale courbée et/ou une extrémité distale biseautée.

13. Sonde selon la revendication 12 en combinaison avec la revendication 10, dans lequel la lentille est agencée à l'extrémité distale du cathéter.

14. Sonde selon la revendication 10, dans laquelle la lentille est agencée sur la circonférence du cathéter.

15. Système d'imagerie comprenant une station échographique et une sonde échographique endoscopique selon l'une des revendications 9 à 14 reliée à ladite station par le connecteur, dans lequel la station comprend un processeur configuré pour traiter une image d'une zone d'intérêt intra-articulaire acquise par la sonde et pour déterminer, à partir de ladite image, au moins une des informations suivantes :
- une épaisseur de cartilage ;
- une rupture du ligament croisé antérieur ;
- un emplacement d'un point d'insertion du ligament croisé antérieur.

## Patentansprüche

1. Hülle (10) für eine endoskopische Ultraschallsonde, umfassend:
- einen ersten flexiblen Abschnitt (10a), der geeignet ist, einen Katheter (4) der Ultraschallsonde zu umgeben, aus einem Material, das geeignet ist, den Ultraschall hindurchzulassen, der ein geschlossenes distales Ende aufweist,
**dadurch gekennzeichnet, dass** die Hülle (10) ferner umfasst
- einen zweiten Abschnitt (10b), der geeignet ist, einen Griff (2) und ein Kabel (3) für eine elektrische Verbindung der Sonde mit einer Ultraschallstation zu umgeben, und
- ein Anschlussstück (11), das zwischen dem ersten und dem zweiten Abschnitt (10a, 10b) angeordnet ist, wobei das Anschlussstück (11) zur lösbaren Befestigung am Griff (2) geeignet ist.

2. Hülle nach Anspruch 1, wobei der erste Abschnitt (10a) aus einem Polyamid-Polyether-BlockCopolymer ist.

3. Hülle nach einem der Ansprüche 1 bis 2, wobei der erste Abschnitt (10a) eine konstante Dicke aufweist.

4. Hülle nach einem der Ansprüche 1 bis 3, wobei die Dicke des ersten Abschnitts (10a) zwischen 0,4 und 0,6 mm liegt.

5. Hülle nach einem der Ansprüche 1 bis 4, wobei der zweite Abschnitt (10b) einen größeren Durchmesser als der erste Abschnitt (10a) aufweist.

6. Hülle nach einem der Ansprüche 1 bis 5, wobei das Anschlussstück eine zentrale Öffnung für den Durchgang des Katheters umfasst, wobei der erste Abschnitt um die zentrale Öffnung des Anschlussstücks herum befestigt ist und der zweite Abschnitt an einem peripheren Abschnitt des Anschlussstücks befestigt ist.

7. Hülle nach einem der Ansprüche 1 bis 6, wobei das Anschlussstück (11) zur Befestigung am Griff (2) durch einen Mechanismus vom Typ Viertelumdrehung geeignet ist.

8. Hülle nach einem der Ansprüche 1 bis 6, wobei das Anschlussstück zur Befestigung am Griff durch Rasten geeignet ist.

9. Endoskopische Ultraschallsonde (100), umfassend:
- einen Ultraschallsensor (5),
- einen Verbinder (1), der zur Verbindung an einer Ultraschallstation geeignet ist,
- einen Steuergriff (2),
- ein elektrisches Verbindungskabel (3), das sich zwischen dem Verbinder (1) und dem Griff (2) erstreckt,
- einen Katheter (4), der einen am Griff (2) befestigten proximalen Abschnitt (2) und einen distalen Abschnitt umfasst, in dem der Ultraschallsensor angeordnet ist,
wobei die Sonde **dadurch gekennzeichnet ist, dass** sie eine Hülle (10) nach einem der Ansprüche 1 bis 8 umfasst, wobei der erste Abschnitt (10a) der Hülle den Katheter (4) umgibt, der zweite Abschnitt (10b) den Griff (2) und das Kabel (3) umgibt und das Anschlussstück (11) lösbar am Griff (2) befestigt ist.

10. Sonde nach Anspruch 9, wobei der Ultraschallsensor einer Linse zugewandt angeordnet ist, die sich auf einer Wand des Katheters befindet.

11. Sonde nach Anspruch 10, wobei der erste Abschnitt (10a) der Hülle den Abschnitt des Katheters bedeckt, in dem der Ultraschallsensor angeordnet ist.

12. Sonde nach einem der Ansprüche 9 bis 11, wobei der Katheter einen gekrümmten distalen Abschnitt und/oder ein abgeschrägtes distales Ende aufweist.

13. Sonde nach Anspruch 12 in Kombination mit Anspruch 10, wobei die Linse am distalen Ende des Katheters angeordnet ist.

14. Sonde nach Anspruch 10, wobei die Linse auf dem Umfang des Katheters angeordnet ist.

15. Bildgebendes System, umfassend eine Ultraschallstation und eine endoskopische Ultraschallsonde nach einem der Ansprüche 9 bis 14, die mit der Station durch den Verbinder verbunden ist, wobei die Station einen Prozessor umfasst, der dazu ausgelegt ist, ein Bild einer intraartikulären interessierenden Zone zu verarbeiten, das von der Sonde erfasst wurde und auf der Basis dieses Bildes mindestens eine der folgenden Informationen zu bestimmen:
- eine Knorpeldicke;
- einen Riss des vorderen Kreuzbands;
- eine Stelle eines Einfügepunkts des vorderen Kreuzbands.

## Claims

1. Sheath (10) for an endoscopic ultrasound probe, comprising :
- a first flexible portion (10a) adapted to surround a catheter (4) of the ultrasound probe, made of a material adapted to allow ultrasound to pass through, having a closed distal end, **characterised in that** said sheath (10) further comprises
- a second portion (10b) adapted to surround a handle (2) and a cable (3) for electrical connection of the probe to an ultrasound station, and
- a connector (11) arranged between the first and second portions (10a, 10b), said connector (11) being adapted to be removably attached to the handle (2).

2. Sheath according to claim 1, wherein the first portion (10a) is made of a polyamide-polyether block copolymer.

3. Sheath according to one of claims 1 to 2, wherein the first portion (10a) has a constant thickness.

4. Sheath according to one of claims 1 to 3, wherein the thickness of the first portion (10a) is between 0.4 and 0.6 mm.

5. Sheath according to one of claims 1 to 4, wherein the second portion (10b) has a diameter greater than that of the first portion (10a).

6. A sheath according to one of claims 1 to 5, wherein the connector comprises a central opening for the passage of the catheter, the first portion being secured around said central opening of the connector and the second portion being secured to a peripheral portion of the connector.

7. Sheath according to one of claims 1 to 6, wherein the connector (11) is adapted to be fixed to the handle (2) by a quarter-turn type mechanism.

8. A sheath according to any one of claims 1 to 6, wherein the fitting is adapted to be secured to the handle by snap-fitting.

9. Endoscopic ultrasound probe (100), comprising :
- an ultrasound sensor (5),
- a connector (1) adapted to be connected to an ultrasound station,
- a control handle (2),
- an electrical connection cable (3) extending between the connector (1) and the handle (2),
- a catheter (4) comprising a proximal portion fixed to the handle (2) and a distal portion in which the ultrasound sensor is arranged,
said probe being **characterised in that** it comprises a sheath (10) according to one of claims 1 to 8, the first portion (10a) of the sheath surrounding the catheter (4), the second portion (10b) surrounding the handle (2) and the cable (3), and the connector (11) being removably fixed to the handle (2).

10. Probe according to claim 9, wherein the ultrasound sensor is arranged opposite a lens located on a wall of the catheter.

11. Probe according to claim 10, wherein the first portion (10a) of the sheath covers the portion of the catheter in which the ultrasound sensor is arranged.

12. A catheter according to any of claims 9 to 11, wherein the catheter has a curved distal portion and/or a bevelled distal end.

13. The probe of claim 12 in combination with claim 10, wherein the lens is arranged at the distal end of the catheter.

14. Probe according to claim 10, wherein the lens is arranged on the circumference of the catheter.

15. Imaging system comprising an echographic station and an endoscopic echographic probe according to one of claims 9 to 14 connected to said station by the connector, wherein the station comprises a processor configured to process an image of an intra-articular zone of interest acquired by the probe and to determine, from said image, at least one of the following items of information:
- cartilage thickness ;
- rupture of the anterior cruciate ligament;
- the location of an insertion point of the anterior cruciate ligament.
